# EUROPEAN PATENT SPECIFICATION

(11) **EP 3 277 339 B1**
(45) Date of publication and mention of the grant of the patent: **18.03.2020**
(21) Application number: 16712906.3
(22) Date of filing: 31.03.2016
(51) Int. Cl.: A61M 1/16, A61M 1/34, A61K 31/194

(54) **A PACKAGE FOR AN ACIDIC DIALYSIS FLUID CONCENTRATE CONTAINING CITRATE AND GLUCOSE**
VERPACKUNG FÜR EIN SAURES DIALYSEFLÜSSIGKEITSKONZENTRAT MIT CITRAT UND GLUCOSE
EMBALLAGE POUR UN CONCENTRÉ DE LIQUIDE DE DIALYSE ACIDE CONTENANT DU CITRATE ET DU GLUCOSE

(30) Priority: 31.03.2015 SE 1550388
(43) Date of publication of application: 07.02.2018
(73) Proprietor: Gambro Lundia AB, 226 43 Lund (SE)
(72) Inventor: MOHAMMED, Husam, SE-215 74 Malmö (SE); WIESLANDER, Anders, SE-227 38 Lund (SE); HANCOCK, Viktoria, SE-241 38 Eslöv (SE); SANDIN, Karin, SE-247 34 Södra Sandby (SE); CARLSSON, Ola, SE-226 49 Lund (SE); LINDEN, Torbjörn, SE-370 23 Hasslö (SE); SZILAGYI, Anna, S-226 48 Lund (SE)
(74) Representative: Ponzellini, Gianmarco
(86) International application number: PCT/EP2016/057077
(87) International publication number: WO 2016/156502

(56) References cited:
- EP-A1- 1 753 437
- EP-A2- 1 101 483
- WO-A1-2014/095953
- JP-B1- 5 517 322
- Anonymous: "Nexcel pharmaceutical films M312, M312A, M312C", , 29 January 2012 (2012-01-29), XP055279685, Retrieved from the Internet: URL:https://web.archive.org/web/2012012917 4149/http://www.sealedairmedical.com/AP/EN /Nexcel_Pharma/M312-312A-312C.aspx [retrieved on 2016-06-10]

## Description

### TECHNICAL FIELD

The present invention relates to a package for an acidic citrate containing concentrate and solution. The present invention does also relate to a system for extracorporeal treatment of blood with the acidic citrate containing concentrate (or the acidic citrate containing solution).

### BACKGROUND OF THE INVENTION

There are a number of various extracorporeal blood treatments, such as hemodialysis, hemofiltration, and hemodiafiltration. They all make use of a filter, also often called dialyzer. A conventional filter comprises a first and a second compartment separated by a membrane, the first compartment having an inlet and an outlet for the circulation of blood and a second compartment having an outlet for draining a liquid (e.g. plasma water, plasma, used dialysis liquid). In case the treatment (e.g. hemodialysis) requires the circulation of a treatment liquid (e.g. a dialysis liquid) the second compartment also has an inlet.

In the above treatments, blood is withdrawn from the patient, flown through the first compartment of the filter, and returned to the patient. This part is called the extracorporeal circuit. In case excess water is to be withdrawn from the blood, this is done across the membrane. In hemodialysis, a dialysis liquid is simultaneously flown through the second compartment of the filter and the metabolic wastes contained in the blood migrate by diffusion through the membrane into the second compartment. In hemofiltration, a pressure difference is created across the membrane so that plasma water flows through the membrane into the second compartment. Metabolic waste migrates by convection into the second compartment. In order to compensate for the loss of body fluid, the patient is simultaneously infused with a substitution solution. Hemodiafiltration is a combination of hemodialysis and hemofiltration. In this treatment a dialysis liquid is flown through the second compartment and a substitution solution is infused into the patient.

The dialysis liquid is thus flown in a dialysis fluid circuit (fluid circuit) from a fluid source, passes the second compartment of the filter, where the blood is treated, and is disposed to a drain.

There are a number of ways to prepare the dialysis fluid and the replacement fluid, commonly called treatment fluid below. Basically different components, often in the form of a concentrate in fluid or powder form, are to be mixed with pure water. Generally the preparation is divided into two main branches. The first one is batch preparation and the second one is on-line preparation. In batch preparation all components that are needed have to be put in the container before the water is added. Thus, the concentration of the different components in relation to each other cannot be changed once the container has been closed by the manufacturer. This document will focus on the on-line preparation.

In on-line preparation of a treatment fluid, the preparation is continuous during the session of blood treatment of a patient. The preparation typically includes feeding of water in a main line and adding the concentrates one after the other along the main line. At the downstream end of the main line, a ready-to-use treatment fluid is outputted. Thus, in the case of a dialysis liquid, the downstream end of the main line is directly connectable to the upstream end of the dialysis fluid circuit (fluid circuit). The concentrates that are being added to the main line are typically in fluid or powder form and are being fed by one pump each. For example, if two concentrates are needed for the dialysis liquid, one may be in fluid form and the other one may be in powder form, or both in powder form.

In chronic dialysis treatment the dialysis fluid is provided by mixing an acidic fluid and a buffered basic fluid to provide a final dialysis fluid being physiologically acceptable with respect to concentration of electrolytes and glucose, as well as pH.

For the final, ready-to-use dialysis fluid there is a broad pH range accepted, a range between 6.9 and 7.6 is considered physiologically acceptable. However, a physiological and most preferred pH of the dialysis fluid is 7.4, and there is an aim to vary only slightly from this pH value for improving the patient compliance and comfort.

The dialysis fluid contains an acidic source for providing the acidic part of an acid/base buffer system to be included in the system.

Historically, the acidic source has comprised acetic acid or salts thereof. In recent years citric acid has emerged as an alternative to acetic acid in dialysis fluids. While increased plasma levels of acetate may induce symptoms like general malaise, intradialytic hypotension and nausea, citrate is a natural source of energy for all cells and part of the acid-base regulation in the body. Citrate has another advantage as it can act as an anticoagulant and antioxidant with an anti-inflammatory property, and may improve patient treatment tolerance. However, it is not only to replace acetic acid with citric acid, but further considerations are needed. Citric acid has specific effect that need to be taken into consideration, namely its (in its citrate form) ability to form a complex with electrolytes within the dialysis fluid. This ability to form complex must be compensated for when deciding on the concentrations of all the components within the dialysis fluid.

Heparin is used as an agent for anticoagulation during dialysis treatment. Most common way of administration is by infusion of heparin, or alternatively as a bolus dose prior the start of the dialysis treatment. However, for some patients, there are drawbacks with heparin infusion like heparin induced thrombocytopenia (HIT) and increased risk of systemic bleeding in the patient. Heparin is commonly used as anticoagulation agent in the hemodialysis methods described above but due to its drawbacks citrate has been discussed as an alternative anticoagulation agent in hemodialysis.

When acetic acid is replaced with citric acid, the result is an acidic concentrate having a pH of between 1 and 2.

However, concentrates and fluids having pH of between 1 and 2 are considered as "strongly acidic", and can cause damages if improper handled. Therefore, there are restrictions and requirements concerning the packaging, labelling, and handling, of the products stated by authorities, and these shall be carefully followed. For example, in 'Guidance for Hazard determination' by U.S. Department of Labor, Occupational Safety and Health Administration, it is explained that in acidic solutions with a pH in the 0 to 2 range cause severe skin and eye burns. Further, in accordance with Canadian Controlled Products Regulations, pH in the range of 0 to 2 is classified as 'strongly acidic'. It is also instructed that materials with pH values of 0 to 2 may be classified corrosive, and shall be stored and handled with great care. Therefore, there is a general desire to avoid acidic solutions having a pH below 2.

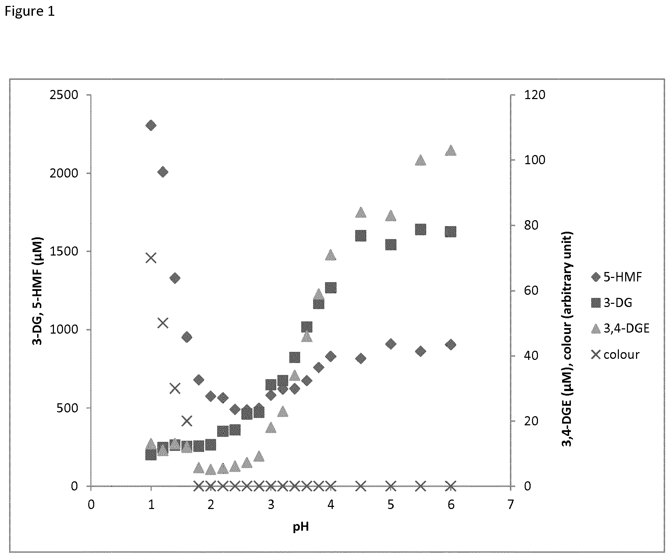

Moreover, many dialysis fluids comprise glucose, and this component is preferably added together with the acidic concentrate. However, it is a challenge to include glucose in an acidic solution due to the potential risk of formation of glucose degradation products (GDPs) such as 5-hydroxymethyl furaldehyde (5-HMF). GDPs are formed at low pH, and also at higher pH value. This has been described for peritoneal dialysis fluid by M. Erixon et al. Peritoneal Dialysis International, Vol 26, pp490-497. This is further shown in Figure 1. Therefore, also from this perspective there is an aim to provide citrate containing acidic concentrates with pH above 2 to minimize glucose degradation and formation of GDPs, for example 5-HMF.

In addition to the GDP formation, glucose containing acidic solutions require special caution due to discoloration of the solution. Discoloration visible for the eye is not acceptable for customers of a fluid intended for use in dialysis treatment. One reason for discoloration can be found in the formation of glucose degradation products during storage. Discoloration can be avoided, or substantially reduced, if the level of oxygen is kept at a desirable level during storage of the acidic dialysis concentrate. Thus, the package containing the glucose containing acidic solution shall allow permeation of oxygen formed during storage.

By providing the citrate containing acidic concentrate in a package of a material having oxygen permeability, especially in a package of a material having oxygen permeability of a certain level, storage stability can be prolonged and discoloration of the product avoided.

Therefore, there is an aim to provide a package containing an acidic citrate containing concentrate, an acidic citrate containing concentrate, and a system wherein acidic citrate containing concentrates having pH above 2 can be used together with bicarbonate containing fluid, and to provide a physiologically acceptable dialysis fluid.

In solutions containing citrate and calcium there is continuously a risk for precipitation of calcium citrate complex. For a solution to be used for preparation of a dialysis fluid the formation of precipitate must be avoided.

In EP 1834652 it is disclosed a dialysis fluid comprising citric acid and/or sodium citrate as pH adjuster. This dialysis fluid keeps the ionized calcium concentration not less than 1 mM.

In JP 2003104869 it is described an agent for dialysis containing citric acid and sodium citrate and is adjusted to pH 2.2-2.9.

WO2014/095953 A1 discloses dialysis fluid compositions comprising citric acid and citrate.

### SUMMARY OF THE INVENTION

The present invention relates to an acidic concentrate package comprising a citrate containing acidic concentrate. The invention is defined in claim 1. Preferred embodiments are specified in the dependent claims.

One object of the present invention is to provide a package including an acidic citrate containing concentrate (may also be denoted 'acidic citrate containing solution') comprising a total concentration of citrate of 28-45 mM; citric acid and citrate in a mole ratio of between 75:25 to 85:15; glucose; and having a pH of between 2 and 3. The package comprises a plastic material having an oxygen permeation rate of more than 2 g/m²/24h at 25°C/90 % RH, for example more than 2.5 g/m²/24h at 25°C/ 90 % RH; or more than 3.5 g/m²/24h at 25°C/90 % RH.

This embodiment has the advantage that it comprises an acidic citrate containing concentrate having a pH of between 2 and 3, to provide a ready to use dialysis fluid having a pH of above 7.25, thus close to physiological pH. With a pH of between 2 and 3, there is a less degree of discoloration due to glucose degradation products (GDPs), in comparison with acidic concentrates having lower or higher pH. A further advantage is that the package comprises an acidic concentrate having a pH above 2, a non-corrosive concentrate, and concentrate not causing any severe skin or eye burns on the users, and not requiring any advanced labelling. Further, by the package, one can also manage to keep a pH closer to physiological while eliminating or reducing the risk of precipitation in the ready-to-use fluid.

In another embodiment of the disclosure, although not forming part of the claimed invention, an acidic citrate containing concentrate is provided. This concentrate is for mixing with a source of bicarbonate, such as a bicarbonate solution, and/or water into a ready to use dialysis fluid. The said acidic citrate containing concentrate comprises a total concentration of citrate of 28-45 mM; citric acid and citrate in a (mole) ratio of between 75:25 to 85:15; has a pH of between 2.1 and 2.4; and said acidic citrate concentrate is sealed in a container having an oxygen permeation rate of more than 2 g/m²/24h at 25 °C/ 90 % RH, for example the oxygen permeation rate is of more than 2.5 g/m²/24h at 25 °C/ 90 % RH, for example more than 3.5 g/m²/24h at 25 °C/ 90 % RH. For example, the acidic citrate containing concentrate comprises the citric acid and citrate in a (mole) ratio of 80:20.

This embodiment has also the advantage that it comprises an acidic citrate containing concentrate having a pH of between 2 and 3, to provide a ready to use dialysis fluid having a pH of above 7.25, thus close to physiological pH. With a pH of between 2.1 and 2.4, there is a less degree of discoloration due to glucose degradation products (GDPs) in comparison with concentrates having pH less than 2, or pH above 3. A further advantage is that the acidic concentrate having a pH of between 2.1 and 2.4 is a non-corrosive concentrate, and a concentrate not causing any severe skin burns or eye damages on the users. In addition, an acidic citrate containing concentrate not showing any precipitation of citrate is provided.

In another embodiment of the invention a system for extracorporeal blood treatment in an extracorporeal blood circuit is provided. The system comprises an arterial blood line configured to be connected to a vascular access for withdrawal of blood from a patient and a venous blood line configured to be connected to the vascular access for returning blood to the patient, and the system comprises:
- a filtration unit with a dialysate side and a blood side, which blood side is in blood communication with the arterial and venous blood lines;
- a source of acidic citrate containing concentrate, which is a package as defined above and in any of claims 1-10;
- a source of bicarbonate (for example as sodium hydrogen bicarbonate); and
which system provides upon mixing of said acidic citrate containing concentrate and said bicarbonate a ready to use dialysis fluid comprising citrate in a total concentration of 0.8-1 mM; 130-150 mM sodium (Na⁺); 20-40 mM bicarbonate; and has a pH above 7.25.

By this embodiment it is possible to provide a dialysis fluid for dialysis treatment which has a pH very close to the physiologically pH, i.e. pH 7.4.

### DEFINITIONS

By the term 'dialysis fluid' it is herein meant the fluid provided on the dialysate side of the semipermeable membrane.

By the term 'filtration unit' it is herein meant the unit comprising one or more semipermeable membranes. In the filtration unit, the blood is flowing on one side of the semipermeable membrane (on the blood side), and the dialysis fluid is flowing on the other side (on the dialysate side). In the filtration unit, the uremic toxins are removed from the blood.

By the term 'acidic citrate containing concentrate' or 'acidic citrate containing solution' it is herein meant an acidic concentrate, or solution, to be combined and mixed with a bicarbonate concentrate to form physiologically acceptable dialysis fluid.

By the term 'total concentration of citrate' it is herein meant the sum of concentration of citric acid and citrate.

By the term 'citrate' it is herein meant any salt form of citric acid. The salt form of citrate may be formed of alkali metals like sodium and potassium, or alternatively, of metals like magnesium and iron. More specifically, when salt is formed with sodium, the citrate may be in form of trisodium citrate, disodium hydrogencitrate, and/or sodium dihydrogen citrate.

### BRIEF DESCRIPTION OF THE DRAWING(S)

Fig. 1 shows the amount glucose degradation products dependent on pH. A glucose solution (50%) was heat sterilized and incubated at 40 degrees C for 30 days. Concentration of 3,4-didexyglucosone-3-ene (3,4-DGE; triangles), 3-deoxyglucosone (3-DG; squares), 5-hydroxymethyl-2 furaldehyde (5-HMF; diamonds) and color (cross; arbitrary unit).

### DETAILED DESCRIPTION OF THE INVENTION

For handling and transporting dialysis fluids, and products related thereto, like acidic concentrate, there are some requirements connected to the package material. For the citrate containing acidic concentrate to be included in the present system there is a desire of package material which reduces the tendency of discoloration of the acidic concentrate.

Further, there are requirements to replace packages, such as plastic containers, of polyvinyl chloride (PVC), commonly used for different solutions used in the field of dialysis treatment. For example, previously, polyvinyl chloride (PVC) was a commonly used material for the plastic containers for acidic concentrates to be used in dialysis treatment. However, this plastic material has low oxygen permeability and when used in plastic containers for acidic concentrates for dialysis fluid, there has been shown discoloration of the product. An alternative plastic material to polyvinyl chloride is polyolefines. However, also features such as permeability of oxygen shall be considered.

The package comprising the acidic citrate containing concentrate is of a plastic material comprising polyolefines, preferably a multilayer film of polyolefine.

The plastic container is made of polyolefines. The group of plastic materials denoted 'polyolefines', or with another name 'polyalkenes' are polymers produced from olefins as the monomer. Examples of polyolefines are polyethylene, polypropylene, etc.

The plastic material providing an oxygen permeation rate as herein defined may be a multilayer film. For example, a suitable plastic material comprises polyolefines with the following structure: a first layer of a modified propylene copolymer, a second layer of polyethylene, and, a third layer of copolyester.

Multilayered films suitable for the acidic citrate containing concentrate are described in EP 0733472 A2 and EP 0738589 A2.

By the present invention, this has surprisingly been solved by having a plastic material of polyolefines having an oxygen permeability of more than 2 g/m²/24h at 25°C/90 % RH.

It has been shown that plastic package material of polyolefines has the property to reduce, or even, avoid the drawbacks above. The package containing an acidic citrate containing concentrate, as herein defined, has a defined oxygen permeation rate. It has been shown that with an oxygen permeation rate of more than 2 g/m²/24h at 25 °C/ 90 % RH; for example more than 2.5 g/m²/24h at 25 °C/ 90 % RH; or more than 3.5 g/m²/24h at 25 °C/ 90 % RH.

The package may, for example, be in form of a bag, such as a bag for 3 to 6 I.

The acidic citrate containing concentrate comprises the citric acid and citrate in a ratio of between 75:25 to 85:15, for example in a ratio of 80:20. The ratio herein considered is the mole ratio of citric acid to citrate.

The package comprises an acidic citrate containing concentrate having a total concentration of citrate of between 28 and 45 mM, for example in a total concentration of citrate of 28, 35 or 45 mM.

The pH of the acidic citrate containing concentrate included in the package is between 2 and 3, for example between 2.2 and 2.4. Examples of suitable pH are 2.2, 2.3 and 2.4.

The acidic citrate containing concentrate included in the package may also comprise further electrolytes and components suitable for dialysis. One such electrolyte is calcium (Ca²⁺). Calcium is present in a concentration that the acidic citrate containing concentrate when diluted to a ready to use dialysis fluid comprises calcium (Ca²⁺) in a concentration of 1.4-1.9 mM. Examples of concentrations of calcium in the ready to use are 1.4, 1.45, 1.5, 1.55, 1.6, 1.65, 1.7, 1.75, 1.8, 1.85, and 1.9 mM.

Another electrolyte to be included in the acidic citrate containing concentrate is potassium. Potassium is present in a concentration that when the acidic citrate containing concentrate is diluted to a ready to use dialysis fluid, the dialysis fluid comprises potassium (K⁺) in a concentration of 0-4 mM, For example, potassium (K+) in the following concentrations 0, 0.5, 1, 1.5, 2, 2.5, 3, 3.5, and 4 mM.

Further, the acidic citrate containing concentrate may also comprise magnesium (Mg²⁺) as an electrolyte. Magnesium is suitable present in a concentration to provide a concentration of between 0.25 mM and 0.75 mM in the diluted acidic citrate containing concentrate, such as 0.5 mM magnesium. For example, magnesium may be present in a concentration of 0.25, 0.3, 0.35, 0.4, 0.45, 0.5, 0.55, 0.6, 0.65, 0.7, and 0.75.

The herein defined package comprises an acidic citrate containing concentrate intended for dilution 1+34, or alternatively 1+44, to form a ready to use dialysis fluid.

The package comprises an acidic citrate containing concentrate having a pH between 2 and 3, for example a pH of between 2.2 and 2.4. For example the pH is 2.2, 2.3, or 2.4.

The package herein described, comprises an acidic citrate containing concentrate which have a composition that when diluted provides a dialysis fluid of the following composition:

| | |
|---|---|
| sodium | 93-113 mM |
| potassium | 0.0-4.0 mM |
| magnesium | 0.25-0.75 mM |
| calcium | 1.25-1.9 mM |
| citric acid | 0.75-0.85 mM |
| citrate | 0.15-0.25 mM |
| chloride | 96-122 |
| glucose | 0-11 mM |

Further, the package herein described, comprises an acidic citrate containing concentrate which have a composition that when diluted provides a dialysis fluid of the following composition:

| | |
|---|---|
| sodium | 93-113 mM |
| potassium | 0.0-4.0 mM |
| magnesium | 0.25-0.75 mM |
| calcium | 1.25-1.9 mM |
| citric acid | 0.75-0.85 mM |
| citrate | 0.15-0.25 mM |
| chloride | 96-122 |
| glucose | 0.1- 11 mM |

More specifically the acidic citrate containing concentrate may have a composition that when diluted provides a dialysis fluid of the following composition:

| | |
|---|---|
| sodium | 93-113 mM |
| potassium | 0.0-4.0 mM |
| magnesium | 0.25-0.75 mM |
| calcium | 1.25-1.9 mM |
| citric acid | 0.80 mM |
| citrate | 0.20 mM |
| chloride | 96-122 |
| glucose | 0-11 mM |

This composition may in another option contain 0.1-11 mM glucose. For example, the composition may comprise glucose in an amount of 5.5 or 5.6 mM.

Depending on the acidity, the low pH, there is a tendency of formation of glucose degradation products. The glucose degradation products include compounds like 5-HMF and FA. Also glucose esters are included in this term. The amount of formed glucose degradation products (GDPs) can be high due to presence of oxygen. Therefore, by having a container of a material having an oxygen permeation, for example, with a rate of more than 2 g/m²/24h at 25 °C/90 % RH the oxygen formed has a possibility to permeate from the solution in a proper way to reduce the amount of glucose degradation products formed, and by that, the discoloration is minimized. Discoloration caused by GDPs has been observed and discussed, for example in 'Singh. B.; Dean. G.R.; Cantor. S.M. The role of 5-(hydroxymethyl)-furfural in the discoloration of sugar solutions. J. Am. Chem. Soc. 1948. 70. 517-522' there is a connection made between discoloration and presence of 5-HMF.

Another embodiment of the disclosure is the acidic citrate containing concentrate for mixing with a source of bicarbonate into a ready to use dialysis fluid. The said acidic citrate containing concentrate comprises a total concentration of citrate of 28-45 mM; citric acid and citrate in a (mole) ratio of between 75:25 to 85:15; has a pH of between 2.1 and 2.4. The said acidic citrate concentrate is sealed in a container having an oxygen permeation rate of more than 2 g/m²/24h at 25°C/ 90 % RH, for example the oxygen permeation rate is of more than 2.5 g/m²/24h at 25°C/90 % RH, for example more than 3.5 g/m²/24h at 25°C/ 90 % RH. For example, the acidic citrate containing concentrate comprises the citric acid and citrate in a (mole) ratio of 80:20. The acidic citrate containing concentrate as above may have a pH of between 2.2 and 2.4.

Another embodiment of the disclosure is a composition, an acidic citrate containing concentrate composition comprising a container and an acidic citrate containing concentrate for mixing with a source of bicarbonate into a ready to use dialysis fluid. The said acidic citrate containing concentrate comprises a total concentration of citrate of 28-45 mM; citric acid and citrate in a (mole) ratio of between 75:25 to 85:15; has a pH of between 2.1 and 2.4. The said acidic citrate concentrate is sealed in said container having an oxygen permeation rate of more than 2 g/m²/24h at 25°C/ 90 % RH, for example the oxygen permeation rate is of more than 2.5 g/m²/24h at 25°C/90 % RH, for example more than 3.5 g/m²/24h at 25°C/ 90 % RH. For example, the acidic citrate containing concentrate comprises the citric acid and citrate in a (mole) ratio of 80:20. The acidic citrate containing concentrate as above may have a pH of between 2.2 and 2.4.

The acidic citrate containing concentrate may, when diluted to a ready to use dialysis fluid, comprise calcium (Ca²⁺) in a concentration of 1.4-1.9 mM.

The acidic citrate containing concentrate when diluted to a ready to use dialysis fluid comprises potassium (K⁺) in a concentration of 0-4 mM

The acidic citrate containing concentrate provided is intended for 1+34; or 1+44 dilution.

A system is also defined herein.The system comprises an arterial blood line configured to be connected to a vascular access for withdrawal of blood from a patient and a venous blood line configured to be connected to the vascular access for returning blood to the patient, and the system comprises:
- a filtration unit with a dialysate side and a blood side, which blood side is in blood communication with the arterial and venous blood lines;
- a source of acidic citrate containing concentrate, said acidic citrate containing concentrate comprises a total concentration of citrate of 28-45 mM; citric acid and citrate in a ratio (mole) of between 75:25 to 85:15; has a pH of between 2 and 3;
- a source of bicarbonate (for example as sodium hydrogen bicarbonate); and
which system provides upon mixing of said acidic citrate containing concentrate and said bicarbonate a ready to use dialysis fluid comprising citrate in a total concentration of 0.8-1 mM; 130-150 mM sodium (Na⁺); 20-40 mM bicarbonate.

Another system provided comprises an arterial blood line configured to be connected to a vascular access for withdrawal of blood from a patient and a venous blood line configured to be connected to the vascular access for returning blood to the patient, and the system comprises:
- a filtration unit with a dialysate side and a blood side, which blood side is in blood communication with the arterial and venous blood lines;
- a source of acidic citrate containing concentrate provided in form of the package as herein defined;
- a source of bicarbonate (for example as sodium hydrogen bicarbonate); and
which system provides upon mixing of said acidic citrate containing concentrate and said bicarbonate a ready to use dialysis fluid comprising citrate in a total concentration of 0.8-1 mM; 130-150 mM sodium (Na⁺); 20-40 mM bicarbonate.

Another system provided comprises an arterial blood line configured to be connected to a vascular access for withdrawal of blood from a patient and a venous blood line configured to be connected to the vascular access for returning blood to the patient, and the system comprises:
- a filtration unit with a dialysate side and a blood side, which blood side is in blood communication with the arterial and venous blood lines;
- a source of acidic citrate containing concentrate provided in form of acidic citrate containing concentrate as herein defined;
- a source of bicarbonate (for example as sodium hydrogen bicarbonate); and
which system provides upon mixing of said acidic citrate containing concentrate and said bicarbonate a ready to use dialysis fluid comprising citrate in a total concentration of 0.8-1 mM; 130-150 mM sodium (Na⁺); 20-40 mM bicarbonate.

By any of these systems, a ready to use dialysis fluid having pH of above 7.25; or pH of between 7.3-7.6, preferably pH of between 7.3-7.4, for example pH of 7.4 is provided.

The system includes an acidic citrate containing concentrate comprising citric acid and citrate in a (mole) ratio of between 75:25 to 85:15, for example, a (mole) ratio of 80:20.

Further, the system provides a ready to use dialysis fluid comprising calcium (Ca²⁺) in a concentration of between 1.4 and 1.9 mM

The system may provide a ready to use dialysis fluid comprising potassium (K⁺), in a concentration of 0-4 mM.

The source of acidic concentrate is intended for 1+34; or 1+44 dilution.

The system herein described, comprises a source of acidic citrate, as the package as herein defined, or as the acidic citrate containing concentrate, thus a composition that when diluted provides a dialysis fluid of the following composition:

| | |
|---|---|
| sodium | 93-113 mM |
| potassium | 0.0-4.0 mM |
| magnesium | 0.25-0.75 mM |
| calcium | 1.25-1.9 mM |
| citric acid | 0.75-0.85 mM |
| citrate | 0.15-0.25 mM |
| chloride | 96-122 |
| glucose | 0-11 mM |

Alternatively, a dialysis fluid of the following composition may be provided:

| | |
|---|---|
| sodium | 93-113 mM |
| potassium | 0.0-4.0 mM |
| magnesium | 0.25-0.75 mM |
| calcium | 1.25-1.9 mM |
| citric acid | 0.75-0.85 mM |
| citrate | 0.15-0.25 mM |
| chloride | 96-122 |
| glucose | 0.1-11 mM |

The system does also include a source of bicarbonate providing a ready to use dialysis fluid comprising citrate in a total concentration of 0.8-1 mM; 130-150 mM sodium (Na⁺); 20-40 mM bicarbonate; and has a pH above 7.25.

In one embodiment the source of citrate containing dialysis concentrate is kept and packaged in a plastic material of polyolefines. The oxygen permeability is defined to be at least 2 g/m²/24h at 25°C/90 % RH, for example 2.5 g/m²/24h at 25°C/90 % RH; 3.5 g/m²/24h at 25°C/90 % RH.

### TEST METHODS AND RESULTS

### EXAMPLE 1

An acidic citrate containing concentrate comprising the following composition:

| | Concentrate | Ready-to-use |
|---|---|---|
| sodium chloride | 4615 mM | 103 mM |
| potassium chloride | 180 mM | 4.0 mM |
| magnesium chloride | 22.5 mM | 0.5 mM |
| calcium chloride | 78.8 mM | 1.75 mM |
| citric acid | 38.3 mM | 0.85 mM |
| citrate | 6.8 mM | 0.15 mM |
| glucose | 250 mM | 5.6 mM |

was prepared. The pH of the concentrate was 2.1.

The acidic concentrate was included in a package comprising a multilayer film of polyolefines (Sealed Air Nexcel® M312A) having an oxygen permeation rate of 3.5 g/m²/day.

The acidic citric containing concentrate was then mixed then mixed with a source of bicarbonate, i.e. a buffered solution comprising bicarbonate in a concentration of 37 mM.

The pH of the final dialysis solution (at 37°C) was estimated to 7.22 (as a theoretical value, not taking in account the carbon dioxide formation).

### EXAMPLE 2

An acidic citric containing concentrate comprising the following composition

| | Concentrate | Ready-to-use |
|---|---|---|
| sodium chloride | 4615 mM | 103 mM |
| potassium chloride | 180 mM | 4.0 mM |
| magnesium chloride | 22.5 mM | 0.5 mM |
| calcium chloride | 78.8 mM | 1.75 mM |
| citric acid | 36.0 mM | 0.80 mM |
| citrate | 9.0 mM | 0.20 mM |
| glucose | 250 mM | 5.6 mM |

was prepared. The pH of the concentrate was 2.2. The pH of the final dialysis solution (at 37°C) was estimated to 7.25 (as a theoretical value).

### EXAMPLE 3

An acidic citrate containing concentrate comprising the following composition:

| | Concentrate | Ready-to-use |
|---|---|---|
| sodium chloride | 4615 mM | 103 mM |
| potassium chloride | 180 mM | 4.0 mM |
| magnesium chloride | 22.5 mM | 0.5 mM |
| calcium chloride | 78.8 mM | 1.75 mM |
| citric acid | 33.8 mM | 0.75 mM |
| citrate | 11.3 mM | 0.25 mM |
| glucose | 250 mM | 5.6 mM |

was prepared in the same way as in Example 1. The pH of the concentrate was measured to pH 2.3. The pH of the final dialysis solution (at 37°C) was estimated to 7.28 (as a theoretical value).

### EXAMPLE 4 - Stability test

The stability of a package comprising the composition of Example 2 was further investigated, following the European Pharmacopeia 8.4 for Haemodialysis solutions, and the test for "Appearance of solution". The multilayer film of the package was of Sealed Air Nexcel® M312A.

The test was performed as a "challenging test", wherein the acidic citrate containing concentrate was kept at 55°C , and 40% RH, during 14 days. The appearance of solution (i.e. the acidic citrate containing concentrate) was checked when test was started, after 3, 7, and 14 days. The solution was not more intensely colored than the reference solution (<Y7).

### EXAMPLE 5 - Test of a package in dialysis machine

A package comprising the acidic citrate containing concentrate of Example 2 was used in a system for extracorporeal blood treatment in an extracorporeal blood circuit as herein defined, as the source of acidic concentrate.

The system is a dialysis machine of model AK 200S, Gambro Lundia AB.

The source of acidic citrate containing concentrate was the concentrate as defined in Example 2; and the source of bicarbonate was provided as BiCart™ (Gambro Lundia AB).

The settings used in the machine were "140/34", thus the dialysis machine provided a dialysis fluid comprising 140 mM sodium, and 34 mM bicarbonate. The pH was measured almost immediately in the ready to use solution by placing the pH electrode in the drain tubing of the dialysis machine. The pH was measured at different time points after the that the dialysis machine gave the "green line". It took 30 minutes (after the machine gave the "green line") to get a stable pH value. pH was measured to 7.31 (measured 30 minutes after "green line").

### Example 6 - Comparative experiment

Example 5 was repeated with a package containing an acidic citrate containing concentrate having a pH of 1.3.
The source of acidic concentrate included citric acid in a concentration to provide 1 mM citric acid/citrate in the ready to use dialysis fluid. The pH of the concentrate in the package was pH 1.3. The same settings as of Example 5 were used, and the pH measurements were made in the same way. pH of the final dialysis solution (at 37°C) was measured to 7.24 (measured 30 minutes after "green line").

Thus, comparison can be made of Examples 1-3 and Example 5, and it can be concluded that a dialysis fluid comprising a pH closer to physiological pH (i.e. pH 7.4) can advantageously be obtained with the package comprising an acidic citrate containing concentrate as herein defined, or with the acidic citrate containing concentrate sealed in the herein specified plastic material.

While the invention has been described in connection with what is presently considered to be the most practical and preferred embodiments, it is to be understood that the invention is not to be limited to the disclosed embodiments. The invention is defined by the appended claims.

## Claims

1. A package including an acidic citrate containing concentrate comprising
a total concentration of citrate of 28-45 mM; citric acid and citrate in a mole ratio of between 75:25 to 85:15; glucose; and having a pH of between 2 and 3; **characterized in that** the package comprises a plastic material having an oxygen permeation rate of more than 2 g/ m²/24h at 25°C/ 90 % RH; preferably more than 2.5 g / m²/ 24h at 25°C/ 90% RH; preferably more than 3.5 g/m²/24h at 25°C/ 90 % RH.

2. The package according to claim 1, wherein acidic citrate containing concentrate have a composition that when diluted provides a dialysis fluid of the following composition:
| | |
|---|---|
| sodium | 93-113 mM |
| potassium | 0-4.0 mM |
| magnesium | 0.25-0.75 mM |
| calcium | 1.25-1.9 mM |
| citric acid | 0.75-0.85 mM |
| citrate | 0.15-0.25 mM |
| chloride | 96-122 mM |
| glucose | 0.1-11 mM |

3. The package according to claim any of claims 1 or 2, wherein acidic citrate containing concentrate comprises the citric acid and citrate in a mole ratio of between 80:20.

4. The package according to claim any of claims 1 to 3, wherein the acidic citrate containing concentrate comprises a total concentration of citrate of 28-45 mM; preferably of 28 mM, 35 mM, or 45 mM.

5. The package according to any of claims 1 to 4, wherein the acidic citrate containing concentrate has a pH of between 2.2 and 2.4, preferably of 2.2, 2.3 or 2.4.

6. The package according to any of claims 1 to 5, wherein the acidic citrate containing concentrate when diluted to a ready to use dialysis fluid comprises calcium (Ca²⁺) in a concentration of 1.4-1.9 mM.

7. The package according to any of claims 1 to 6, wherein the acidic citrate containing concentrate when diluted to a ready to use dialysis fluid comprises potassium (K+) in a concentration of 0-4 mM.

8. The package according to any of claims 1 to 7, wherein it is of plastic material comprising polyolefines, preferably is a multilayer film of polyolefine.

9. The package according to any of claims 1 to 8, wherein the acidic citrate containing solution is intended for dilution 1+34 to form a ready to use dialysis fluid.

10. The package according to any of claims 1 to 8, wherein the acidic citrate containing solution is intended for dilution 1+44 to form a ready to use dialysis fluid.

11. System for extracorporeal blood treatment in an extracorporeal blood circuit comprising an arterial blood line configured to be connected to a vascular access for withdrawing blood from a patient and a venous blood line configured to be connected to the vascular access for returning blood to the patient, the system comprising:
- a filtration unit with a dialysate side and a blood side, which blood side is in blood communication with the arterial and venous blood lines;
- a source of acidic citrate containing concentrate, which is a package as defined in any of claims 1 to 10; and
- a source of bicarbonate;
and which system is configured to provide upon mixing of said acidic citrate containing concentrate and said bicarbonate a ready to use dialysis fluid comprising citrate in a total concentration of 0.8-1 mM;130-150 mM sodium (Na); 20-40 mM bicarbonate .

12. System according to claim 11, wherein the ready to use dialysis fluid has a pH of above 7.25, a pH of between 7.3-7.6, preferably a pH of between 7.3-7.4.

13. The system according to any of claims 11 to 12, wherein the ready to use dialysis fluid further comprises calcium (Ca²⁺) in a concentration of 1.4-1.9 mM.

14. The system according to any of claims 11 to 13, wherein the ready to use dialysis fluid further comprises potassium (K⁺) in a concentration of 0-4 mM.

15. The system according to any of claims 11 to 14, wherein the source of acidic concentrate is intended for 1+34 or 1+44 dilution.

## Patentansprüche

1. Packung, die ein saures Citrat enthaltendes Konzentrat mit einer Gesamtkonzentration an Citrat von 28-45 mM,
Citronensäure und Citrat in einem Molverhältnis von 75:25 bis 85:15,
Glucose und
mit einem pH-Wert zwischen 2 und 3
einschließt, **dadurch gekennzeichnet, dass** die Packung ein Kunststoffmaterial mit einer Sauerstoffpermeationsrate von mehr als 2 g/m²/24 h bei 25°C/90% RF, vorzugsweise mehr als 2,5 g/m²/24 h bei 25°C/90% RF, vorzugsweise mehr als 3,5 g/m²/24 h bei 25°C/90% RF.

2. Packung nach Anspruch 1, wobei das saures Citrat enthaltende Konzentrat eine Zusammensetzung aufweist, die verdünnt eine Dialyseflüssigkeit der folgenden Zusammensetzung bereitstellt:
| | |
|---|---|
| Natrium | 93-113 mM |
| Kalium | 0-4,0 mM |
| Magnesium | 0,25-0,75 mM |
| Calcium | 1,25-1,9 mM |
| Citronensäure | 0,75-0,85 mM |
| Citrat | 0,15-0,25 mM |
| Chlorid | 96-122 mM |
| Glucose | 0,1-11 mM. |

3. Packung nach einem der Ansprüche 1 oder 2, wobei das saures Citrat enthaltende Konzentrat die Citronensäure und das Citrat in einem Molverhältnis von zwischen 80:20 umfasst.

4. Packung nach einem der Ansprüche 1 bis 3, wobei das saures Citrat enthaltende Konzentrat eine Gesamtkonzentration an Citrat von 28-45 mM, vorzugsweise von 28 mM, 35 mM oder 45 mM, umfasst.

5. Packung nach einem der Ansprüche 1 bis 4, wobei das saures Citrat enthaltende Konzentrat einen pH-Wert zwischen 2,2 und 2,4, vorzugsweise von 2,2, 2,3 oder 2,4, aufweist.

6. Packung nach einem der Ansprüche 1 bis 5, wobei das saures Citrat enthaltende Konzentrat verdünnt zu einer gebrauchsfertigen Dialyseflüssigkeit Calcium (Ca²⁺) in einer Konzentration von 1,4-1,9 mM umfasst.

7. Packung nach einem der Ansprüche 1 bis 6, wobei das saures Citrat enthaltende Konzentrat verdünnt zu einer gebrauchsfertigen Dialyseflüssigkeit Kalium (K⁺) in einer Konzentration von 0-4 mM umfasst.

8. Packung nach einem der Ansprüche 1 bis 7, welche aus einem Polyolefine umfassenden Kunststoffmaterial besteht, und bei dem es sich vorzugsweise um einen mehrlagigen Polyolefinfilm handelt.

9. Packung nach einem der Ansprüche 1 bis 8, wobei die saures Citrat enthaltende Lösung für die Verdünnung 1+34 unter Bildung einer gebrauchsfertigen Dialyseflüssigkeit vorgesehen ist.

10. Packung nach einem der Ansprüche 1 bis 8, wobei die saures Citrat enthaltende Lösung für die Verdünnung 1+44 unter Bildung einer gebrauchsfertigen Dialyseflüssigkeit vorgesehen ist.

11. System zur extrakorporalen Blutbehandlung in einem extrakorporalen Blutkreislauf mit einer Leitung für arterielles Blut, die so konfiguriert ist, dass sie mit einem Gefäßzugang zur Entnahme von Blut aus einem Patienten verbunden ist, und eine Leitung für venöses Blut, die so konfiguriert ist, dass sie mit dem Gefäßzugang zur Rückführung des Bluts zu dem Patienten verbunden ist, wobei das System Folgendes umfasst:
- eine Filtrationseinheit mit einer Dialysatseite und einer Blutseite, wobei die Blutseite sich in einer Blutverbindung mit den Leitungen für arterielles und venöses Blut befindet,
- eine Quelle von saures Citrat enthaltendem Konzentrat, bei dem es sich um eine wie in einem der Ansprüche 1 bis 10 definierte Packung handelt, und
- eine Hydrogencarbonatquelle,
und wobei das System so konfiguriert ist, dass es beim Mixen des saures Citrat enthaltenden Konzentrats und des Hydrogencarbonats eine gebrauchsfähige Dialyseflüssigkeit liefert, die Citrat in einer Gesamtkonzentration von 0,8-1 mM, 130-150 mM Natrium (Na) und 20-40 mM Hydrogencarbonat umfasst.

12. System nach Anspruch 11, wobei die gebrauchsfertige Dialyseflüssigkeit einen pH-Wert von über 7,25, einen pH-Wert zwischen 7,3-7,6 und vorzugsweise einen pH-Wert zwischen 7,3-7,4 aufweist.

13. System nach einem der Ansprüche 11 bis 12, wobei die gebrauchsfertige Dialyseflüssigkeit weiterhin Calcium (Ca²⁺) in einer Konzentration von 1,4-1,9 mM umfasst.

14. System nach einem der Ansprüche 11 bis 13, wobei die gebrauchsfertige Dialyseflüssigkeit weiterhin Kalium (K⁺) in einer Konzentration von 0-4 mM umfasst.

15. System nach einem der Ansprüche 11 bis 14, wobei die Quelle von saurem Konzentrat für eine 1+34- oder 1+44-Verdünnung vorgesehen ist.

## Revendications

1. Emballage comportant un concentré contenant du citrate acide, comprenant une concentration totale en citrate de 28-45 mM ;
de l'acide citrique et du citrate selon un rapport molaire allant de 75:25 à 85:15 ;
du glucose ; et
ayant un pH compris entre 2 et 3 ;
**caractérisé en ce que** l'emballage comprend une matière plastique ayant un taux de perméation de l'oxygène supérieur à 2 g/m²/24 h à 25°C/90% de HR ; préférablement supérieur à 2,5 g/m²/24 h à 25°C/90% de HR ; préférablement supérieur à 3,5 g/m²/24 h à 25°C/90% de HR.

2. Emballage selon la revendication 1, dans lequel le concentré contenant du citrate acide possède une composition qui, lors d'une dilution, fournit un liquide de dialyse ayant la composition suivante :
| | |
|---|---|
| sodium | 93-113 mM |
| potassium | 0-4,0 mM |
| magnésium | 0,25-0,75 mM |
| calcium | 1,25-1,9 mM |
| acide citrique | 0,75-0,85 mM |
| citrate | 0,15-0,25 mM |
| chlorure | 96-122 mM |
| glucose | 0,1-11 mM. |

3. Emballage selon la revendication l'une quelconque des revendications 1 ou 2, dans lequel le concentré contenant du citrate acide comprend l'acide citrique et le citrate selon un rapport molaire de 80:20.

4. Emballage selon la revendication l'une quelconque des revendications 1 à 3, dans lequel le concentré contenant du citrate acide comprend une concentration totale en citrate de 28-45 mM ; préférablement de 28 mM, 35 mM, ou 45 mM.

5. Emballage selon l'une quelconque des revendications 1 à 4, dans lequel le concentré contenant du citrate acide possède un pH compris entre 2,2 et 2,4, préférablement de 2,2, 2,3 ou 2,4.

6. Emballage selon l'une quelconque des revendications 1 à 5, dans lequel le concentré contenant du citrate acide, lors d'une dilution en un liquide de dialyse prêt à l'emploi, comprend du calcium (Ca²⁺) selon une concentration de 1,4-1,9 mM.

7. Emballage selon l'une quelconque des revendications 1 à 6, dans lequel le concentré contenant du citrate acide, lors d'une dilution en un liquide de dialyse prêt à l'emploi, comprend du potassium (K⁺) selon une concentration de 0-4 mM.

8. Emballage selon l'une quelconque des revendications 1 à 7, où il s'agit d'une matière plastique comprenant des polyoléfines, préférablement il s'agit d'un film multicouche de polyoléfine.

9. Emballage selon l'une quelconque des revendications 1 à 8, dans lequel la solution contenant du citrate acide est prévue pour une dilution à 1+34 afin de former un liquide de dialyse prêt à l'emploi.

10. Emballage selon l'une quelconque des revendications 1 à 8, dans lequel la solution contenant du citrate acide est prévue pour une dilution à 1+44 afin de former un liquide de dialyse prêt à l'emploi.

11. Système destiné à un traitement sanguin extra-corporel dans un circuit sanguin extra-corporel comprenant un conduit de sang artériel configuré pour être connecté à un accès vasculaire afin de prélever du sang à partir d'un patient et un conduit de sang veineux configuré pour être connecté à l'accès vasculaire afin de renvoyer le sang vers le patient, le système comprenant :
- une unité de filtration avec un côté dialysat et un côté sang, lequel côté sang est en communication sanguine avec les conduits de sang artériel et veineux ;
- une source de concentré contenant du citrate acide, qui est un emballage tel que défini selon l'une quelconque des revendications 1 à 10 ; et
- une source de bicarbonate ;
et lequel système étant configuré pour fournir, lors du mélange dudit concentré contenant du citrate acide et dudit bicarbonate, un liquide de dialyse prêt à l'emploi comprenant du citrate selon une concentration totale de 0,8-1 mM ; 130-150 mM de sodium (Na) ; 20-40 mM de bicarbonate.

12. Système selon la revendication 11, dans lequel le liquide de dialyse prêt à l'emploi possède un pH supérieur à 7,25, un pH de 7,3-7,6, préférablement un pH de 7,3-7,4.

13. Système selon l'une quelconque des revendications 11 à 12, dans lequel le liquide de dialyse prêt à l'emploi comprend en outre du calcium (Ca²⁺) selon une concentration de 1,4-1,9 mM.

14. Système selon l'une quelconque des revendications 11 à 13, dans lequel le liquide de dialyse prêt à l'emploi comprend en outre du potassium (K⁺) selon une concentration de 0-4 mM.

15. Système selon l'une quelconque des revendications 11 à 14, dans lequel la source de concentré acide est prévue pour une dilution à 1+34 ou 1+44.
